# FASCICULE DE BREVET EUROPEEN

(11) **EP 0 487 433 B1**
(45) Date de publication et mention de la délivrance du brevet: **11.10.1995**
(21) Numéro de dépôt: 91420408.6
(22) Date de dépôt: 18.11.1991
(51) Int. Cl.: A61F 2/36, A61F 2/46

(54) **Prothèse fémorale partiellement cimentée**
Teilweise zementierte Femurprothese
Partially cemented femoral prosthesis

(30) Priorité: 19.11.1990 FR 9014694
(43) Date de publication de la demande: 27.05.1992
(73) Titulaire: TORNIER SA, F-38330 Saint Ismier (FR)
(72) Inventeur: Tornier, Alain, F-38190 Crolles (FR)
(74) Mandataire: Karmin, Roger

(56) Documents cités:
- EP-A- 0 083 028
- EP-A- 0 357 546
- EP-A- 0 393 425
- DE-A- 3 937 786
- US-A- 4 881 536
- US-A- 4 888 022

## Description

La présente invention concerne un élément fémoral pour une prothèse totale de hanche et plus particulièrement ses moyens de fixation dans le canal médullaire du fémur.

On sait qu'à ce jour, il existe trois procédés pour la fixation des endoprothèses d'articulation de la hanche, à savoir soit par assujettissement à l'aide d'un ciment, soit par impaction directe, c'est-à-dire l'engagement à force contrôlée de la tige fémorale dans le canal médulaire sans utilisation de ciment, et soit par fixation partielle et uniquement dans la partie supérieure du canal médullaire comme décrit dans le brevet US 4 888 022.

Pour les prothèses fémorales fixées par l'intermédiaire d'un ciment, il existe trois méthodes de mise en oeuvre :
- mettre simplement le ciment acrylique dans le canal médullaire et impacter la prothèse ;
- placer un bouchon dans le canal médullaire à environ 1 cm au-delà de l'extrémité de la tige fémorale, puis déverser ou injecter du ciment dans le canal et enfin impacter la tige afin de mettre sous pression le ciment préalablement introduit, dont la quantité excessive ressort par l'extrémité supérieure du fémur.
- introduire une prothèse dont la tige est creuse et solidaire à son extrémité libre d'un joint se développant lors de l'injection du ciment dans ladite tige pour éviter au ciment de pénétrer plus profondément dans le canal médullaire comme décrit dans le brevet DE-39 37 786.

Ces prothèses cimentées comportent certains inconvénients en ce qui concerne les risques liés à l'utilisation du ciment et plus particulièrement dûs à l'élévation de la température et à une libération de produits qui présentent une toxicité variable.

On obtient évidemment une fixation immédiate, mais sur toute la longueur de la tige sans zone privilégiée, ne prenant pas en compte les différentes zones d'élasticité du fémur.

Cette zone de fixation change la transmission naturelle des contraintes sur l'os et modifie à long terme les structures osseuses dans le sens de la détérioration de la fixation.

En cas de reprise, il faut enlever tout le ciment et pour cela percer le bouchon de ciment à l'extrémité inférieure de la prothèse, ce qui est un travail long et difficile.

Les prothèses fémorales fixées sans ciment comportent un aspect de surface sur toute ou partie de la tige fémorale, de manière à obtenir une stabilité immédiate dans le canal médullaire.

Cette méthode de fixation comporte certains inconvénients du fait de la forme en dépouille des tiges des prothèses, entraînant un contact très aléatoire en partie métaphysaire du fémur et limité dans les meilleurs cas à quelques zones qui ne peuvent être prévues. De plus, l'os supporte des contraintes lors de la fixation et une détérioration importante lorsque la prothèse fémorale doit être retirée.

Enfin, il peut se produire une micromobilité entraînant à moyen terme une séparation.

Le dernier procédé de fixation consiste en une prothèse de hanche dont la partie supérieure est recouverte sur la totalité de sa surface d'une enveloppe élastomère. Cette enveloppe forme une poche de retenue lors de l'introduction d'un fluide durcissable. Elle se gonfle pour venir plaquer contre les parois internes du canal médullaire et plus particulièrement dans la zone diaphyso-métaphysaire du fémur. Enfin, la surface externe de l'enveloppe comporte des zones de réhabilitation de l'os qui sont disposées suivant un quadrillage déterminé.

Cette méthode de fixation comporte certains inconvénients du fait des contraintes opératoires qu'engendre l'introduction d'un élément nouveau à l'intérieur du canal médullaire. Du plus, l'enveloppe élastomère risque de ne pas être étanche dans sa partie inférieure sous l'effet de pression lors de l'introduction du fluide durcissable. Enfin, la mise en place de cette enveloppe sur le corps de la prothèse de hanche augmente de manière non négligeable son prix de revient.

Les perfectionnements qui font l'objet de la présente invention visent à remédier aux inconvénients précités et à permettre la réalisation d'une prothèse de hanche dont la tige fémorale ne soit cimentée que dans sa partie supérieure, la partie inférieure non cimentée déterminant par un profil approprié un guidage axial acceptable et facile de la prothèse.

A cet effet, la tige de la prothèse suivant l'invention comprend dans sa partie médiane de l'élément, c'est-à-dire dans la zone diaphyso-métaphysaire du fémur, un joint d'étanchéité destiné à retenir un ciment acrylique qui est introduit jusqu'à débordement dans la partie supérieure du canal médullaire.

Le dessin annexé, donné à titre d'exemple, permettra de mieux comprendre l'invention, les caractéristiques qu'elle présente et les avantages qu'elle est susceptible de procurer :
Fig. 1 est une coupe longitudinale montrant la prothèse fémorale suivant l'invention, introduite et scellée dans le canal médullaire d'un fémur ;
Fig. 2 est une vue de côté partielle avec arrachement illustrant le positionnement du joint qui retient le ciment ;
Fig. 3 est une vue de face partielle ;
On a représenté en fig. 1 l'élément fémoral 1 d'une prothèse de hanche et qui est destiné à être placé à l'intérieur du canal médullaire 21 d'un fémur 2.

L'élément 1 comprend une tige 10 et un embout incliné 13 supportant à son extrémité libre une tête ou une boule céphalique 12 illustrée en traits interrompus et réalisant avec la prothèse cotyloïdienne l'articulation du fémur par rapport à l'os iliaque.

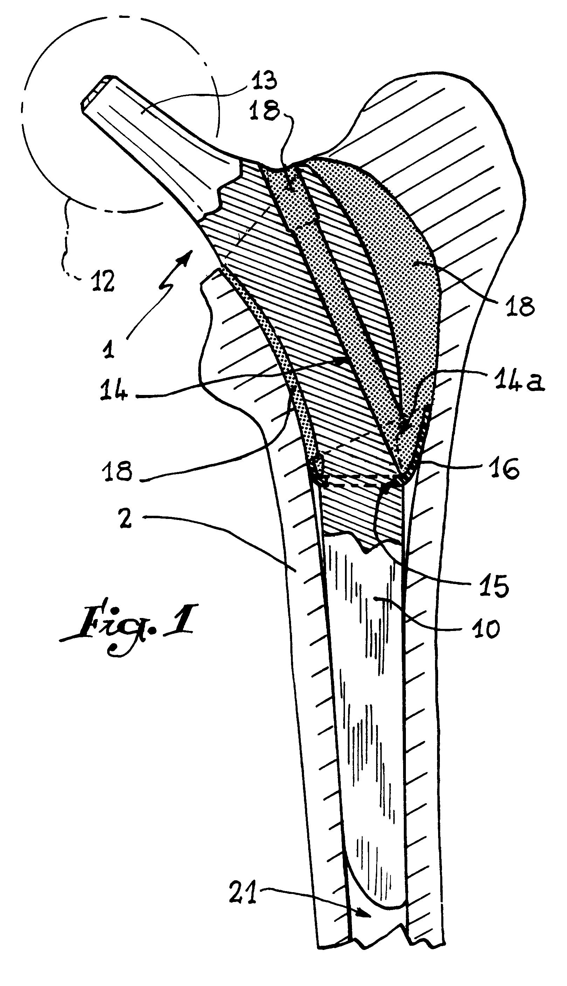

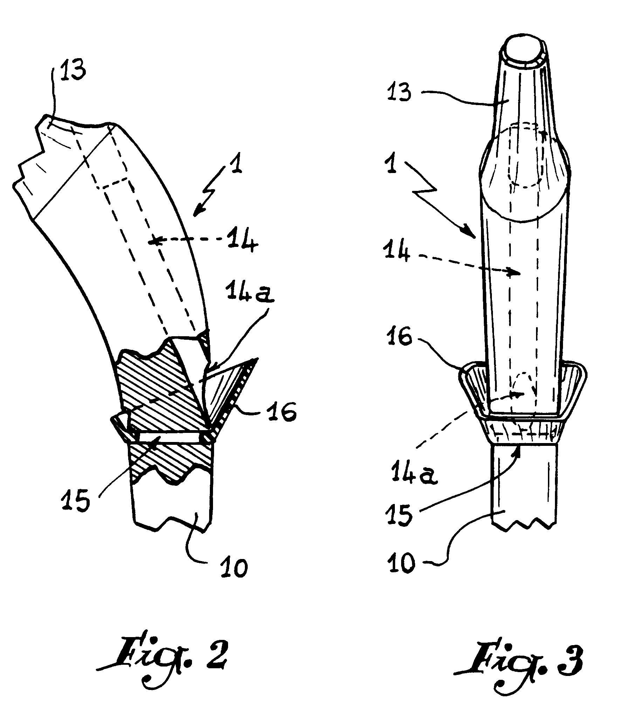

La tige 10 de l'élément 1 est pourvue par exemple d'un alésage oblique 14 d'inclinaison quelconque la traversant de part en part de manière à venir déboucher dans la zone médiane de ladite tige et à l'opposé de l'embout 13, comme représenté en fig. 1 et 2.

Conformément à l'invention, on pratique suivant la morphologie du patient par exemple dans la partie médiane de la périphérie de la tige 10 une gorge 15 disposée juste en dessous de l'extention débouchante la plus basse de l'alésage oblique 14 (fig. 2 et 3). La gorge 15 permet la mise en place d'un joint 16 en forme de cuvette ou de collerette dont la concavité est tournée en direction de l'embout incliné 13. Ce joint est asymétrique, c'est-à-dire que sa partie oblique la plus haute se trouve du côté de l'extrémité débouchante 14a la plus basse de l'alésage 14 (fig. 2 et 3).

Lors de la mise en place de la tige fémorale 10 à l'intérieur du canal médullaire 21, le joint est comprimé dans le sens centripète et vient contre la paroi dudit canal. Une fois la tige en position définitive dans ce dernier, le joint 16 réalise une étanchéité entre la partie supérieure et la partie inférieure du canal médullaire 21 du fémur 2.

La fixation de l'élément fémoral 1 est obtenue par introduction d'un ciment acrylique 18 à l'intérieur de l'alésage oblique 14, permettant d'amener ledit ciment en contact sous pression avec le joint 16, de sorte que ce dernier se gonfle jusqu'à venir se plaquer énergiquement contre les parois du canal médullaire 21. On remplit alors la cavité déterminée au-dessus du joint 16 entre la prothèse et le canal médullaire 21 du fémur 2 avec le ciment acrylique 18 jusqu'à ce qu'il déborde à la partie supérieure dudit canal, et qu'il reflue par l'extrémité supérieure de l'alésage oblique 14.

A cet effet, tout autre moyen pourrait être utilisé pour l'injection du ciment acrylique 18 directement dans la partie supérieure du canal médullaire 21, par exemple une seringue adaptée, l'alésage 14 étant alors inutile.

Le joint de retenue 16 peut être réalisé soit en une matière silastique qui, du fait de sa composition, s'adapte parfaitement aux parois du canal médullaire 21, soit en polyéthylène ou en une matière biorésorbable.

Cette méthode de fixation partielle permet d'obtenir une prothèse 1 ayant une stabilité axiale parfaite du fait que la partie inférieure de la tige 10 est placée sans ciment 18 dans la partie correspondante du canal médullaire 21, tandis que les quelques degrés de liberté de la tige dans la partie supérieure dudit canal sont éliminés par l'intermédiaire du ciment 18 qui assure une fixation solide et immédiate de la prothèse.

Le fait de ne remplir le canal médullaire 21 que dans sa partie supérieure autour de la tige 10 réduit nettement la quantité de ciment et diminue les risques de toxicité et d'échauffement. De même, on obtient grâce à l'invention une très grande amélioration du temps de travail lors de la révision d'une prothèse, puisque l'alimentation du ciment 18 au moment du retrait de la prothèse fémorale s'effectue très facilement une fois la tige fémorale retirée.

Il va de soi que l'on peut remplacer les gorges 15 permettant la mise en place du joint de retenue 16, par tout autre moyen par exemple par coincement ou collage sans pour cela changer la portée de l'invention.

## Revendications

1. Prothèse de la hanche du genre comprenant une tige fémorale pourvue d'un embout incliné recevant la boule céphalique fixée partiellement uniquement et plus particulièrement dans la partie supérieure du canal médullaire et comportant un alésage oblique (14) dont l'origine se trouve à la jonction de l'embout (13) par rapport à la tige fémorale et qui débouche au niveau de la partie médiane de l'élément (10), dans la zone diaphyso-métaphysaire du fémur, caractérisée en ce qu'elle comporte dans sa partie médiane un joint d'étanchéité ou joint de retenue (16) asymétrique présentant un profil en forme de cuvette dont la concavité est tournée en direction de l'embout incliné (13) joint destiné à retenir un ciment acrylique (18) qui est introduit dans l'alésage oblique (14) jusqu'à débordement dans la partie supérieure du canal médullaire (21) et des moyens de retenue du joint d'étanchéité situés en dessous de l'extrémité débouchante (14a) de l'alésage (14).

2. Prothèse suivant la revendication 1, caractérisée en ce que a partie oblique la plus haute joint de retenue (16) asymétrique, se trouve du côté de l'extrémité débouchante (14a) la plus basse de l'alésage (14).

3. Prothèse fémorale suivant l'une quelconque des revendications 1 et 2, caractérisée en ce que le joint de retenue (16) est fixé sur la tige fémorale (1) par l'intermédiaire d'une gorge (15) ménagée sur la périphérie de l'élément (10).

4. Prothèse suivant l'une quelconque des revendications 1 et 2, caractérisée en ce que le joint de retenue (16) est fixé sur l'élément (10) soit par collage, soit par coïncement.

5. Prothèse fémorale suivant l'une quelconque des revendications précédentes, caractérisée en ce que le joint de retenue (16) est réalisé en une matière plastique ou bio-résorbable.

## Claims

1. Hip prosthesis of the type comprising a femoral stem provided with an inclined tip receiving the cephalic ball partially fixed only and more particularly in the upper part of the medullary cavity, and including an oblique bore (14), the origin of which is located at the junction of the tip (13) with the femoral stem and which emerges in the median part of the element (10), in the diaphysometaphyseal zone of the femur, characterized in that it includes, in its median part, an asymmetric sealing joint or retention joint (16) having a cup-shaped profile, the concavity of which is turned towards the inclined tip (13), which joint is intended to retain an acrylic cement (18) which is introduced into the oblique bore (14) until it protrudes into the upper part of the medullary cavity (21) and sealing joint retention means located below the emergent end (14a) of the bore (14).

2. Prosthesis according to Claim 1, characterized in that the highest oblique part of the asymmetrical retention joint (16) is located on the side of the lowest emergent end (14a) of the bore (14).

3. Femoral prosthesis according to either of Claims 1 and 2, characterized in that the retention joint (16) is fixed on the femoral stem (1) by means of a groove (15) arranged on the periphery of the element (10).

4. Prosthesis according to either of Claims 1 and 2, characterized in that the retention joint (16) is fixed onto the element (10) either by adhesive bonding or by wedging.

5. Femoral prosthesis according to any one of the preceding claims, characterized in that the retention joint (16) is made of a plastic or bioabsorbable material.

## Patentansprüche

1. Hüftprothese von der Art, die einen Femurschaft aufweist, der mit einem den Kugelkopf aufnehmenden, geneigten Ansatz versehen ist, die nur teilweise und inbesondere im oberen Bereich des Medullarkanals befestigt ist und eine Schrägbohrung (14) aufweist, deren Ursprung sich am Übergangsbereich vom Ansatz (13) zum Femurschaft befindet, und die auf Höhe des mittleren Bereichs des Elements (10) im Diaphyse-Metaphysebereich des Femurs mündet,
dadurch **gekennzeichnet**,
daß sie in ihrem mittleren Bereich eine asymmetrische Abdichtungs- oder Auffangeinrichtung (16) aufweist, die ein Profil in Form eines Napfes aufweist, dessen Hohlbereich in Richtung des geneigten Ansatzes (13) ausgerichtet ist, wobei die Dichtung dem Auffangen eines Acrylzements (18) dient, der in die Schrägbohrung (14) eingebracht wird, bis er über den oberen Bereich des Medullarkanals (21) fließt, und daß sie Mittel zum Halten der Abdichtung aufweist, die unterhalb des mündenden Endes (14a) der Bohrung (14) gelegen sind.

2. Prothese nach Anspruch 1,
dadurch **gekennzeichnet**,
daß der oberste schräge Bereich der asymmetrischen Auffangeinrichtung (16) sich neben dem untersten mündenden Ende (14a) der Bohrung (14) befindet.

3. Femurprothese nach Anspruch 1 oder 2,
dadurch **gekennzeichnet**,
daß die Auffangeinrichtung (16) auf dem Femurschaft (1) über eine Nut (15) befestigt ist, die am Umfang des Elements (10) vorgesehen ist.

4. Prothese nach einem der Ansprüche 1 oder 2,
dadurch **gekennzeichnet**,
daß die Auffangeinrichtung (16) auf dem Element (10) entweder durch Kleben oder durch Pressen befestigt ist.

5. Femurprothese nach einem der vorhergehenden Ansprüche,
dadurch **gekennzeichnet**,
daß die Auffangeinrichtung (16) aus einem Kunststoff oder einem bioresorbierbaren Material hergestellt ist.
